# EUROPEAN PATENT APPLICATION

(11) **EP 0 631 766 A1**
(43) Date of publication of application: **04.01.1995**
(21) Application number: 93305097.3
(22) Date of filing: 29.06.1993
(51) Int. Cl.: A61F 13/15

(54) **Disposable garments and method for the manufacture thereof**

(71) Applicant: Herrin, Robert M., Orlando, Florida 32810 (US); Tharpe, John M., Albany, Georgia 31707 (US)
(72) Inventor: Herrin, Robert M., Orlando, Florida 32810 (US); Tharpe, John M., Albany, Georgia 31707 (US)
(74) Representative: Robinson, Nigel Alexander Julian

(57) **Abstract**

Disposable garments of the training pants variety include a cuff (80) which is rolled inwardly from the plane of each leg opening (56). The rolled cuff feature (80) is obtained during production by stretching a unitary elastic sheet (40) across the machine web (34) in a direction lateral to the direction of manufacture, and then cutting leg openings (56) through the elastic sheet (40) and an adjacent outer web (34). The machinery for manufacturing the garments includes various features which facilitate the manufacture of this garment, including a reciprocating cutting knife and an associated accumulating conveyor for providing a reciprocal motion into the reciprocal knife. The manufacturing apparatus also includes features for maintaining core pads in the desired alignment, and various other features.

## Description

The present invention relates to disposable garments and methods and apparatus for making such garments.

The prior art teaches a wide variety of disposable diaper configurations, in which the finished diaper is a flat panel having adhesive tabs or the like, permitting the joinder of one end of the panel to the other, to fit the diaper panel from the infant's back, under the crotch and to the front of the waist. Examples of such arrangements are shown in the following U.S. Patents: 3,081,772 to Brooks et al; 3,417,751 to Murdoch; 4,892,528 to Suzuki et al; and 5,064,489 to Ujimoto et al.

There have also been developed in the prior art a variation of finished disposable training pants for infants, in which the finished product has opposing side seams joining the front and back panels of the garment assembly. Examples of these arrangements are shown in the following U.S. Patents: 4,743,239 to Cole; 4,743,241 to Igaue et al; 4,646,362 to Heran et al; 4,938,757 to Van Gompel et al; and 5,055,103 to Nomura et al; and 5,064,421 to Tracy.

The aforementioned U.S. Patent 4,743,241 to Igaue et al discloses an elastic sheet adhered across the panel adjacent to the opposing leg openings, which serves as a liquid barrier for body fluids leaking from the center absorbent pad. A similar arrangement is disclosed in U.S. Patent 3,417,751 to Murdoch.

The present invention is directed to disposable garments, particularly of the training pants variety, which include a rolled cuff inwardly from the plane of each leg opening, at least about the lower portion of the leg opening. Further, the present invention is directed to methods and apparatus for achieving this rolled cuff feature in a low cost and easy manner. The present invention also has the objective of providing methods and apparatus for achieving improvements and efficiencies in the manufacture of disposable garments of all types, including both disposable diapers, training pants, and incontinent garments for those who are elderly or infirmed.

In one form, a disposable garment in accordance with the present invention includes a panel of a material at least a portion of which is liquid impervious, the panel defining leg openings. The garment is provided with elastic means extending continuously about the peripheral edge of each leg opening with the elastic means including a sheet of an elastic material stretched along an inside surface of the panel and about a substantial portion of the periphery of the corresponding leg opening, the elastic sheet being under sufficient stretch to draw the periphery of the leg opening into a curved elastic cuff when the garment is fitted about an infant. In this arrangement, the elastic sheet extends continuously around and to the peripheral edge of the corresponding leg opening.

In another form, the elastic sheet extends about the lower portion of the leg opening, with plural elastic bands extending about the remaining periphery of the leg opening at the upper portion on either side of the leg opening. In this arrangement, the elastic bands preferably also extend across the area covered by the corresponding elastic sheet, then laterally across the crotch area of the panel and then across a portion of the area covered by another elastic sheet about the opposing leg opening, with a similar pair of elastic bands being also disposed around the other side of the two leg openings.

A third form of construction for the disposable garment employs a second layer of elastic sheet extending between the leg openings, and longitudinally stretched between the leg holes. The second stretched elastic layer avoids the necessity for providing the elastic bands of the type described above, while at the same time providing a uniform gathering of material in the crotch area of the garment.

Preferably, the elastic sheet material is under a stretch on the order of five ounces per lineal inch for each inch of cross-width when installed across the panel, and extends at least 1.27 cms (0.5 in.) from the peripheral edge laterally across that portion of the panel where the elastic sheet is deposited.

In the manufacture of the garments, a unitary elastic sheet is stretched across the machine web in a direction lateral to the direction of manufacture, the elastic sheet having dimensions so as to cover an area of the web where leg openings for two adjacent garments will later be formed. Leg openings are then cut through each elastic sheet in the outer web, each leg opening lying within the boundaries of the corresponding elastic sheet and defining the peripheral edges of two leg openings, one each for the adjacent garments. The web is then later folded in half, so that the elastic sheet extends continuously around the leg opening. In this form of manufacture, the direction of stretch of the elastic sheet for each leg opening thus is generally parallel to the edge of the opening along the lower portion, the direction of stretch extending somewhat laterally away from the peripheral edge along that portion closest to the waist opening of the garment. Appropriate seams are formed along the sides between the front and back of the garment from the waist opening to the upper portion of each leg opening.

The present invention also is directed to manufacturing methods and apparatus, which includes a sequential series of operations formed at individual stations, or "sections", where the method and apparatus coact with the materials which make up the disposable garments.

One of the features of the present invention with respect to methods and apparatus for manufacturing such garments is the inclusion of a reciprocating "guillotine" cutting knife for severing the continuous web of absorbent material into individual pads. The use of a reciprocating knife reduces the hardness and sharpness of the edge of the pad, with respect to conventional rotary cutters. In order to accommodate the use of the reciprocating cutting blade, the present invention utilizes a conveying system in which the web of materials forming the pads are passed into an accumulator section which permits an elevated continuous feed from overhead onto a vacuum conveyor; the vacuum conveyor is then operated intermittently as required by the operation of the reciprocating cutting knife. The accumulator utilizes a retaining flap to keep the degree of accumulating "bend" in the continuous pad material to an acceptable level. The material forming the pad web is maintained against the vacuum conveyor with a curved bar extending in the direction of manufacture; the vacuum conveyor itself utilizes a woven material as the conveyor surface, with a fixed vacuum plate for drawing the pad web down against the woven conveyor surface. The reciprocating action of the knife across the laterally extending pad web is operated by an eccentric drive mechanism, and the cutting aperture below the reciprocating knife blade is angled in a V-shape, in order to facilitate a smooth cut.

A second feature of the present invention is located downstream of the reciprocal cutter. After cutting of the individual pads, the pads are then conveyed to a combining station and adhered to an outer carrier layer, or web. In order to insure that the pads are delivered exactly in a straight format to the combining station, the pad conveyor between the cutter and the combining section is provided with means for straightening any misaligned pads moving across the conveyor; this objective is achieved in a conveyor which also further achieves the objective of reaching near the throat of the reciprocal cutting knife, in order to receive each cut pad. This is achieved using multiple thin, plastic webbing strips for the conveyor surface, and a chain and dog drive positioned underneath the webbing conveyor, the chain having an eccentric drive to move each dog first slowly, then faster, then slowly again. The chain drives extend parallel with the thin webbing, with the dogs extending through the webbing and into engagement with each pad. In operation, each straightening dog rises slowly between two of the conveyor webbing strips, then speeds up to engage the backside of a corresponding pad on opposite sides of the webbing, thereby straightening any misaligned pad. As the pad then nears the discharge end of the webbing conveyor, the eccentric drive of the chain assembly slows down each dog relative to the speed of the webbing conveyor, thereby causing each dog to effectively back away from the respective pad; each dog then is rotated below the level of the webbing conveyor.

Yet another feature of the method and apparatus relates to techniques for folding of an outer non-woven layer over the inner layers. In a preferred arrangement, the web making up the disposable garment includes an outer non-woven layer having a polyvinyl liquid impervious liner adhered to the outer non-woven layer, with the absorbent pad adhered to the liner, and with an inner non-woven layer adhered across the pad and the liquid impervious liner. The elastic sheet is disposed and adhered to across the inner surface of the outer non-woven layer, before disposition of the liquid impervious liner across the outer non-woven layer. Waistband elastic may also be adhered along the edges of the outer non-woven layer before fixing the polyvinyl barrier liner. Preferably, the outer non-woven layer has a lateral dimension relative to the direction of manufacture which is somewhat greater than the other layers, so that an edge portion of the outer layer extends beyond the other layers. This outer edge portion is then folded inwardly across the waistband elastic and the edges of the polyvinyl liner and then inner non-woven layer. This folded edge provides further comfort for the person wearing the garment, and also protects the waistband edge. This folding is achieved in a facile manner according to the present invention utilizing a feed roller having beveled ends, the dimensions for which correspond generally to the outwardly extending edge portion of the outer non-woven layer. These beveled ends permit the relaxation of the outer edge portion as the machine web passes underneath the roller under tension; thereafter, edge folding fixtures extend underneath and complete the folding of the edge portions with an adhesive. Of course, this edge folding function is achieved downstream from the combining section, where the absorbent pad is joined with the outer non-woven layer and the polyvinyl liner, and after the inner non-woven layer is affixed across the liner.

Following the edge folding operation, the machine web is passed into a leg hole cutting section, where leg holes are cut through the inner and outer non-woven layers, the polyvinyl liner and centrally through the elastic sheets. The dimensions of the elastic sheet are such as to extend outside of the leg hole dimensioned on the order of at least 1.27 cms (0.5 in.), or more, as discussed above. Further, it will be appreciated that each leg hole defines a corresponding leg opening for adjacent garments which are eventually severed from the machine web. It is preferred that the leg hole cutting operation take place with a vacuum underneath the cutter, in order to draw away the waste product of that cutting operation.

Following the cutting of the leg holes, the machine web is then folded in half and passed into a seam weld section, where two adjacent seams are formed using an ultrasonic welder. In one arrangement, the anvil of the ultrasonic welder is heated to a temperature somewhat below the melting temperature of the polyvinyl liner, and the teeth on the anvil are sharpened to obtain penetration through all of the layers. The combination of the sharpened anvil teeth and the elevated temperature provide a highly reliable seam weld.

A second form of the ultrasonic seam welder utilizes an inflatable bladder behind the anvil, with the ultrasonic horn driven by an eccentric cam. These features permit a greater dwell time during the seaming operation.

The products are then passed into a unit cutter section, where the machine web is severed into individual garments.

These and other features of the present invention will be understood by those skilled in the art from the following detailed description and the accompanying drawings in which:
FIGURES 1A and 1B are flow charts illustrating the system, method and apparatus of the present invention, as used in the manufacture of disposal garments.
FIGURES 2A and 2B are top and front views, respectively, illustrating the component portions and manner of fabrication of disposable garments in accordance with the present invention and in which various locations along the direction of operation in FIGURES 2A and 2B generally correspond to the location of the various sections of the system illustrated in FIGURES 1A and 1B.
FIGURE 3 is a side elevation, partially in cross section, of a disposable garment in accordance with the present invention.
FIGURE 4 is a cross section of a portion of the disposable garment shown in FIGURE 3, taken along the line 4-4.
FIGURE 5 is another cross section of a portion of the disposable garment shown in FIGURE 3, taken along the line 5-5.
FIGURE 6A is a top view of a second form of a disposable garment in accordance with the present invention.
FIGURE 6B is a top view of a third form of a disposable garment according to this invention.
FIGURE 7 is a side elevation illustrating the accumulator section of the system shown in FIGURE 1A.
FIGURE 8 is front elevation and FIGURE 9 is a side elevation, both illustrating the features of the reciprocal knife cutting section of the system of FIGURE 1A.
FIGURE 10 is a top plan view of the pad conveyor section of the system shown in FIGURE 1A.
FIGURE 11 is a cross section of a portion of the conveyor shown in FIGURE 10, taken along the lines 11-11.
FIGURES 12 and 13 are top and side views, respectively, of the stretcher and applicator section of the system shown in FIGURE 1A.
FIGURE 14 is a front view of a portion of the edge-folding section in the system shown in FIGURE 1A, and FIGURE 15 is top view of that edge folding section.
FIGURE 16 is a perspective view illustrating one form of the seam welding section of the system in FIGURE 1B.
FIGURES 17A and B are top and side views illustrating construction details for a second form of the seam welding section.
FIGURE 17C is a diagram illustrating the movement of the horn and anvil in the construction of FIGURES 17A and B.

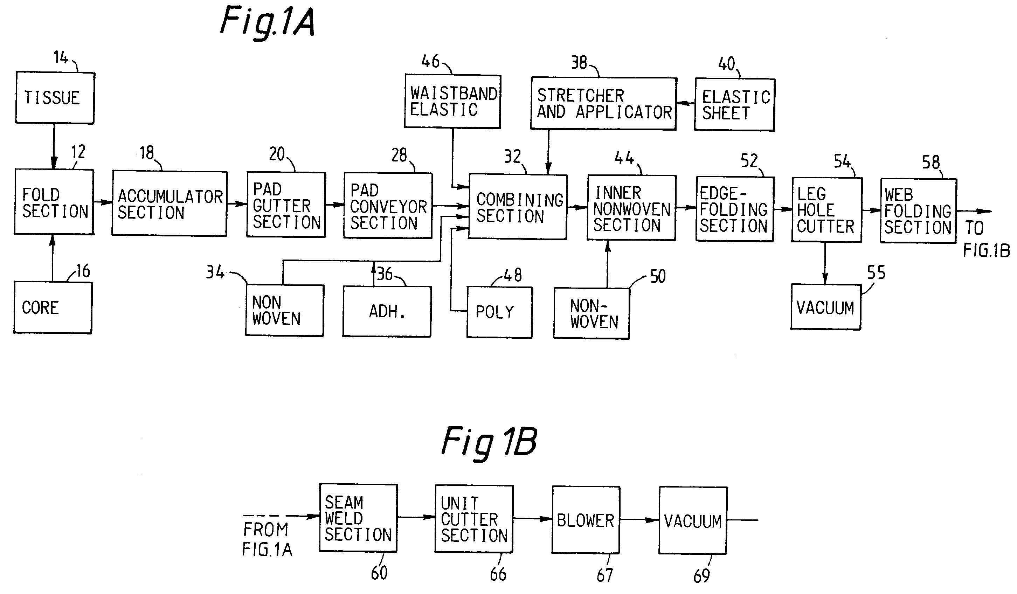

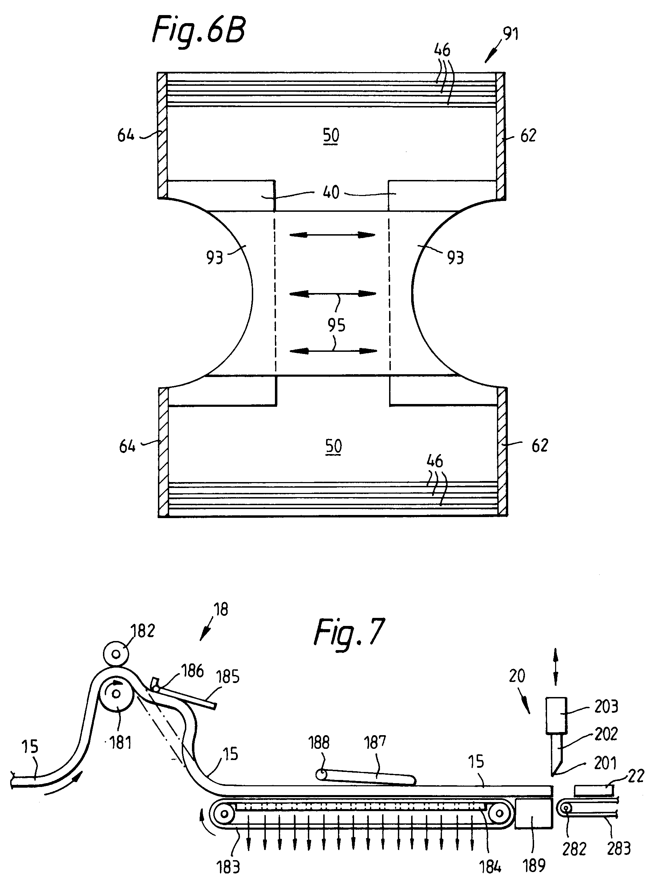

In Figures 7-16, three digit reference numerals are used to identify specific features of a corresponding portion of the system shown in Figures 1A and 1B, and in which the reference numeral for the section in Figures 1A and 1B has the same first two digit reference numeral (for example, in Figure 7, three digit reference numerals 181, 182 and so forth refer to specific features of the accumulator section 18 in Figure 1A).

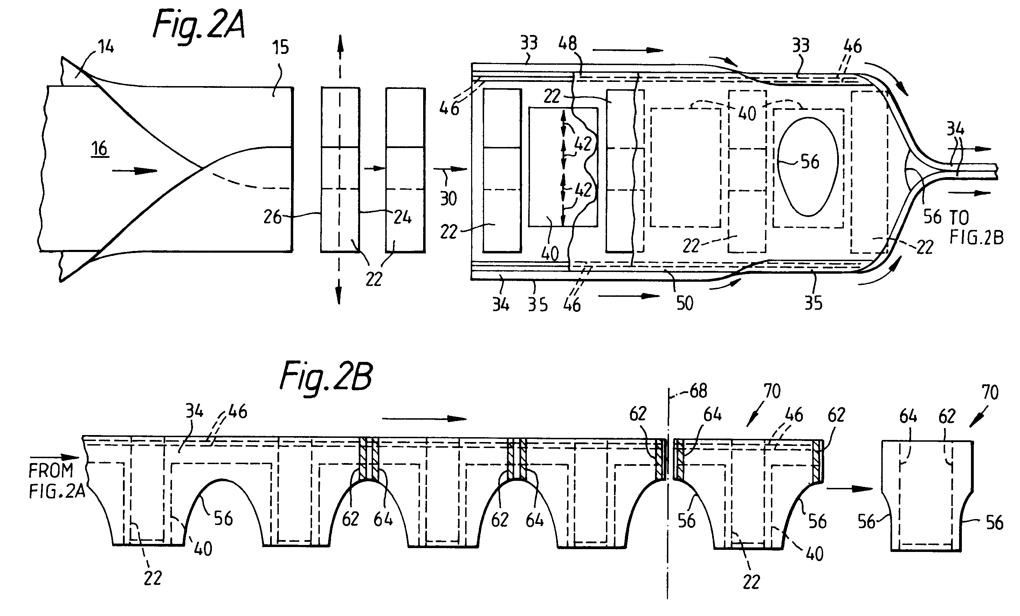

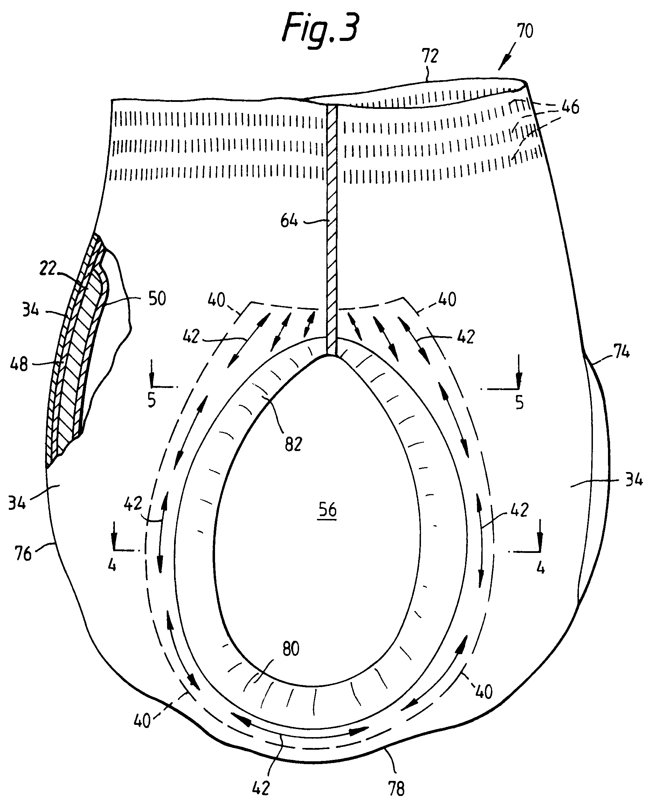

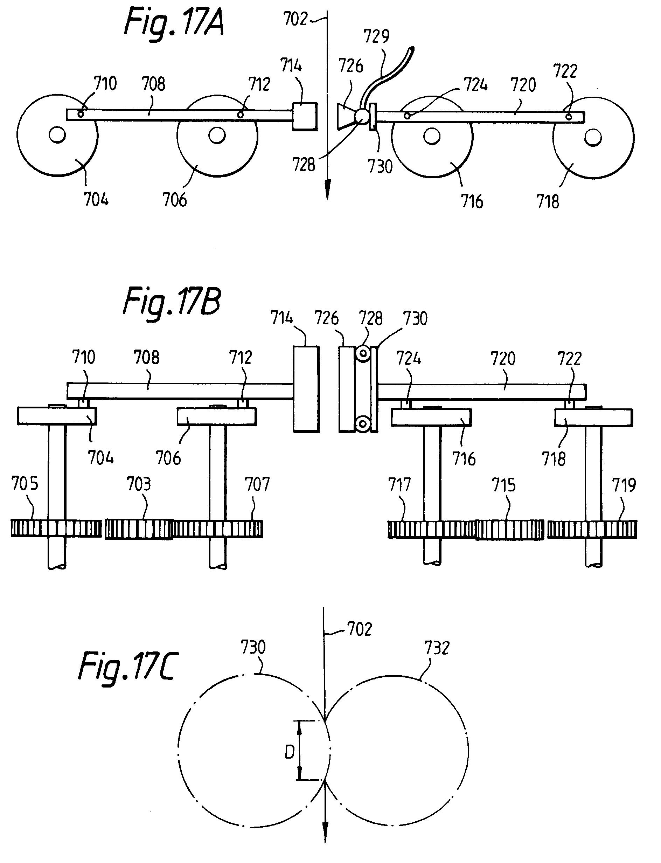

Referring to the drawings, method and apparatus of this invention for making disposable garments will first be described with reference to Figures 1A and 1B, with concurrent reference to Figures 2A and 2B. Thereafter, three forms of disposable garments made according to the method and using the apparatus Figures 3-6A and B, followed by a description of specific features of the apparatus as shown in Figures 7-16, and an alternate seam weld construction in Figures 17A-C..

Referring to Figures 1A and 2A, the apparatus 10 includes a fold section 12 using conventional techniques to fold an absorbent core material 16 and an outer tissue 14, in order to form a web of absorbent pad material 15. The output of the fold section is passed into an accumulator section 18, which is shown schematically in Figure 1A to be elevated with respect to the fold section 12, and the downstream pad cutter section 20. As illustrated in Figure 2A, the absorbent pad web 15 is cut into individual pads 22, each having a forward edge 24 and a rear edge 26, by cutting along a direction lateral to the direction of manufacture, as illustrated by L-30. The individually cut pads 22 are passed into a pad conveyor section 28, and then to a combining section 32.

In the combining section 32, an outer non-woven layer 34 is extended along the direction of manufacture 30, and provided with an appropriate adhesive, generally over the entire exposed surface of the non-woven layer from 34. Waistband elastic strips 46 are extended along the adhesive-coated non-woven outer layer 34, again in the direction of manufacture 30, in order to provide a waistband elastic in a conventional manner. A liquid impervious liner 48 (for example, polyvinyl) is extended across the adhesive-coated non-woven layer 34, and then individual pads 22 are affixed to the exposed surface of the liner, using appropriate adhesives.

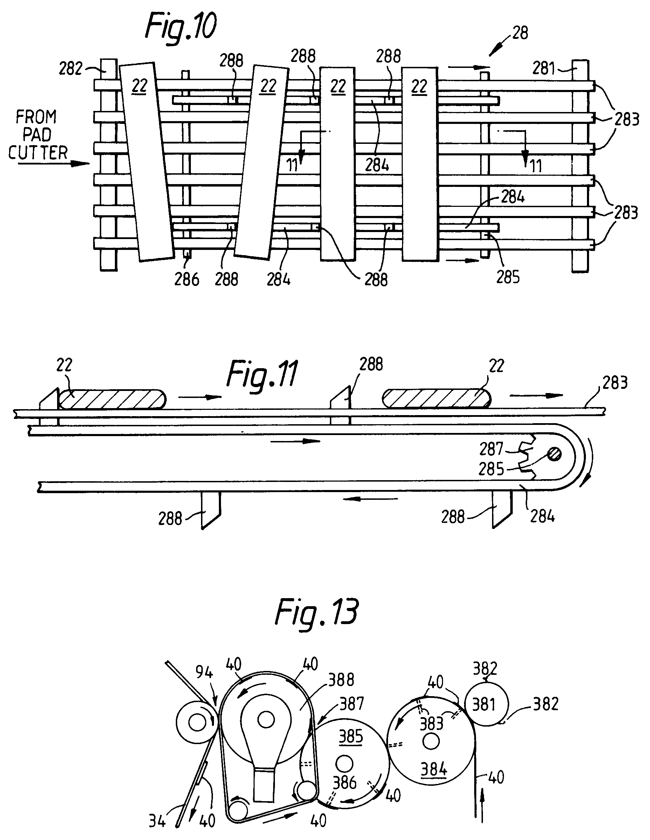

In accordance with the present invention, there is provided a source of elastic sheet material 40, for example polyurethane sheeting or polyethylene form, which is extended into a stretcher and applicator section 38, which is described in greater detail below with reference to Figures 12 and 13. As shown in Figure 2A, the stretcher and applicator 38 cuts the elastic sheet into individual sheet segments 40, while simultaneously stretching each sheet segment 40 in a direction lateral to the direction of manufacture 30, as is depicted by arrows 42 in Figure 2A, and generally under the stretch conditions discussed above, that is to say a stretch on the order of 55.86 g per cm (5 oz. per lined inch) for each inch (2.54 cms) of cross-width i.e., 709 g (25 ozs.) for a 12.7 cms (5 ins.) length of one inch (2.54 cms) wide elastic). The individual elastic sheets segments 40 may be affixed to either the liquid impervious liner 48, or to the outer non-woven layer 34, as shown in Figure 2A. The polyvinyl liner 48 and the individual pads 22 are respectively affixed to the outer non-woven layer 34 and the liner 48 with a conventional adhesive 36.

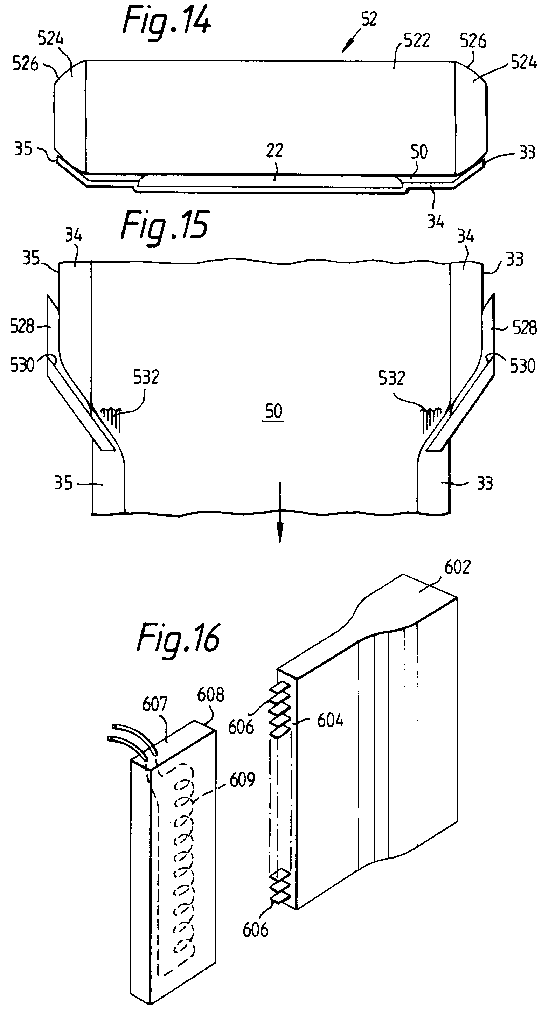

The output of the combining section is fed into another section where an inner non-woven layer 50 is fixed to the assembly by an adhesive across the liner 48. As is shown in Figure 2A, the inner non-woven layer 50 and the liner 48 have a lateral dimension somewhat less than the corresponding dimension of the outer non-woven layer 34, so that the outer non-woven layer has peripheral edges 33, 35 extending beyond the periphery of the other two layers. After deposition of the inner non-woven layer at section 44, the assembly is then fed into an edge folding section 52. As schematically depicted in Figure 2A, the peripheral edges 33, 35 of the outer non-woven layer 34 are then folded across the periphery of the inner non-woven layer 50 and joined thereto with an adhesive, as described in greater detail below with reference to Figures 14 and 15.

The output from the edge folding section 52 is then fed into a leg hole cutting section 54, which is operated under a slight vacuum at 55, so as to remove the scrap without interfering with the manufacture of the assembly. Noting Figure 2A, each leg hole 56 is cut centrally in the web defined by the layers 34, 48 and 50 between adjacent pads 22, and centrally within the peripheral boundary of a corresponding elastic sheet 40; preferably, the dimensions of the leg hole and the elastic sheet are selected so that a minimum of 1.27 cms (0.5 in.) of the elastic sheet, and suitably an even greater dimension, extend from the peripheral edge of the leg hole 56 to the boundary of the elastic sheet 40. It will of course be appreciated that the cutting of the leg hole 56 centrally in the elastic sheet 40 insures that the elastic sheet extends to the very edge of the leg hole. As will be discussed in greater detail below with reference to Figures 3-5, this particular feature enables the disposable garment to achieve a desirable inwardly rolled cuff which assists in defining a liquid barrier.

After passing out of the leg hole cutting section 54, the assembly is fed into a web folding section 58, where the entire web is folded upon itself, as depicted along the left hand side of Figure 2B. The web is then fed into a seam welding section 60, where parallel seams are formed between the upper edge of the web and the upper extremity of each leg hole 56; preferably, this is achieved utilizing an ultrasonic welder and a rotating anvil which is heated to a temperature slightly below the melting point of the polyvinyl liner 48, and below the ignition temperature of the non-woven layers 34 and 50.

The output from the seam welding section is then passed into a unit cutter section 66, where the web is cut into individual garments 70. Each garment is then conveyed into a blower section 67, where air is blown into the individual garment, following which each individual garment is subjected to a vacuum at 69 to draw the intermediate seam welds inwardly to form a compact unit for packaging purposes.

Construction details of a first form of the disposable garment 70 are depicted in Figures 3-5. As constructed, the garment 70 includes a waistband opening 72, a front panel 74, an opposing rear panel 76 and a crotch area 78; it will of course be understood that the front and back panels and the crotch area 74, 76 and 78 are formed from the assembly of materials in accordance with the method depicted in Figures 2A and 2B, and includes (as shown in the cut away portion in Figure 3) the outer non-woven layer 34, the liner 48, absorbent pad 22 and inner non-woven layer 50, with the elastic waistbands 46 and seam 64.

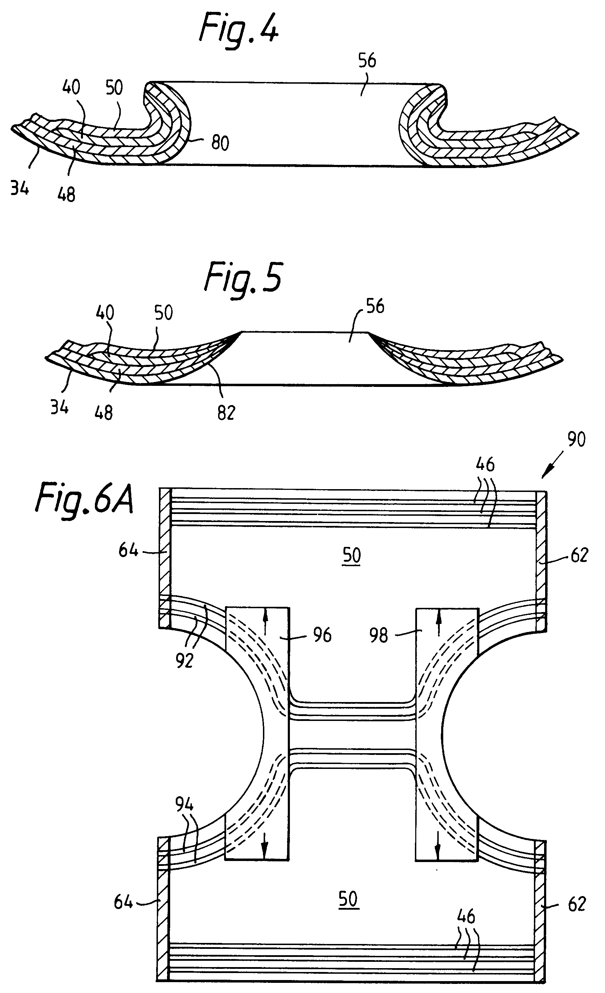

The portion of the elastic sheet 40 remaining with the individual garment 70 after formation of the leg opening 56, folding of the web and welding of the seam 64 is depicted by a dotted line in Figure 3. Arrows 42 also depict the direction of stretch of the elastic sheet 40. As discussed above, the cutting of the leg opening 56 through the field of the elastic sheet 40 to extend to the extreme periphery of the opening 56. This, together with the folding of the web to form the front and back panels 74, 76 relieves the stretching of the elastic sheet 40 along the periphery of the leg opening 56, and causes the elastic sheet along the periphery to roll into an inwardly-directed cuff along the periphery of the leg opening 56. However, because of the variance of the stretch represented by arrows 42 from the lower portion 80 of the leg opening 56 to the upper portion 82, the degree of roll of the cuff varies from the lower portion to the upper portion. This is shown by comparison of Figures 4 and 5, where the lower portion 80 of the rolled cuff is shown to be greater than the upper portion 82 of Figure 5, this variance of the rolled cuff from the lower portion is desirable, since the upper portion does not require as great a roll in the cuff for purposes of providing a liquid barrier, and also provides greater comfort to the person wearing the garment 70.

A second alternate form of construction of the disposable garment in accordance with the present invention is shown in Figure 6A and referred to generally by the reference numeral 90.

Disposable garment 90 includes various features of the garment 70 shown in Figures 3-5, which are referred to by the same reference numerals, including waistband elastic 46, seams 62, 64, inner non-woven layer 50, pad 22 and leg openings 56. The construction of disposable garment 90 from the construction of garment 70 in Figure 3 through the use of elastic bands 92 and 94 which extend continuously around the periphery of opposite sides of each leg opening and across a portion of the same area covered by elastic sheets 96, 98 and also across the crotch area of the garment 90 to the opposite leg opening 56. Further, the elastic sheets 96, 98 may be drawn back slightly from the respective seams 64, 62 so as to extend essentially about the peripheral edge of the lower portion of the leg opening 56 after being folded.

A third form of construction of a disposable garment in accordance with the present invention is shown in Figure 6B and referred to there by the reference numeral 91.

The disposable garment 91 also includes the various features of the garments 70 and 90 shown in Figures 3-5 and 6A, to the extent that those various features are referred to by the same reference numerals. Additionally, the garment 91 includes a second layer of elastic sheeting extending between the leg openings, and longitudinally stretched in the direction of the arrows 95 in Figure 6B. The stretched elastic layer 93 avoids the necessity for providing the elastic bands 92 and 94 of Figure 6A, while at the same time providing a uniform gathering of material in the crotch area of the garment 91.

Details of the accumulator section 18 of Figure 1A are shown in Figure 7.

As shown on the left hand side of Figure 7, the absorbent pad web 15 is extended across elevated rollers 181, 182 which operate continuously to receive the feed of the web 15. The rollers 181, 182 then feed the web 15 downwardly across an indexing conveyor 183, which preferably consists of a wire mesh extending across a fixed vacuum plate 184 having holes therein permitting a vacuum to be drawn, as shown by arrows. the accumulator section 18 further includes a control plate 185 pivoted at 186, and extending across the web 15 somewhat below the continuous feed rollers 181, 182. Similarly, there is a pressure rod 187 pivoted at 188 extending across the absorbent pad web 15 and over the indexing conveyor 183.

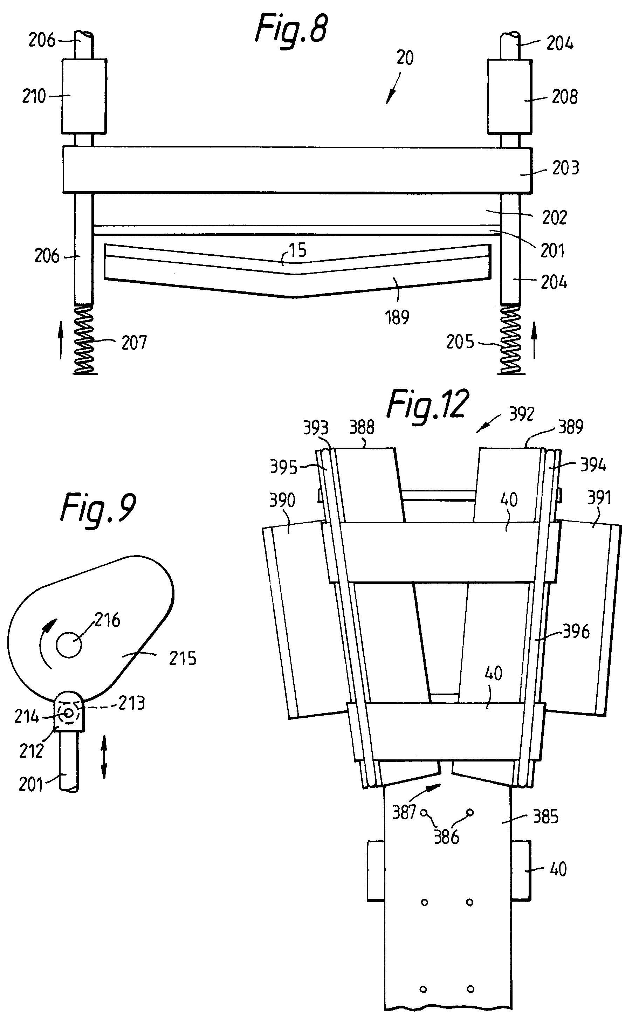

In operation, the absorbent pad web 15 is drawn into fixed engagement with the mesh surface of the indexing conveyor 183 via the vacuum plate 184. The indexing conveyor 183 is operated on a "stop-start", or intermittent basis, as required by the operation of the reciprocating cutting blade 20 described below in greater detail with reference to Figures 8 and 9, but the details of which are also depicted in Figure 7. Thus, the rollers 181 and 182 are continuously feeding the web 15 onto a conveyor which only operates on an intermittent basis; therefore, the web 15 is subjected to a buckling, as shown below the control plate 185. The degree of buckle is controlled by the plate 185, to insure that the direction of the web continues along the surface of the intermittent indexing conveyor 183. The pressure rod 187 also insures that any buckling does not extend beyond the central area of the indexing conveyor 183.

As shown on the right hand side of Figure 7, the indexing conveyor 183 forces the absorbent pad web 15 across a fixed mandrel 189 in the throat of the cutting section 20, described next.

As discussed briefly above, the pad cutting section 20 utilizes a reciprocating "guillotine"-type of cutter, which achieves certain benefits with respect to the formation of the cut edge in the absorbent pads 22.

Referring to Figure 8, the cutting section 20 includes a blade 202 terminating in a cutting edge 201, and carried by a cross plate 203 extending across the machine direction. The plate 203 is carried by reciprocating rods 204, 206 which are supported by springs 205, 207 for biasing in an upward direction. The reciprocating rods 204, 206 are supported by respective bearing blocks 208, 210. As shown in the front view of Figure 8, the mandrel 189 has a V-configuration, such that the absorbent pad web 15 is sheared downwardly from each side to the central portion of the mandrel.

Noting Figure 9, the reciprocating motion of the rods 204, 206 is controlled by an eccentric 215 rotating about a shaft 216, and engaging a rotating element 213 mounted on a pivot axis 214 and which is supported by a yoke 212 at the upper extremity of each rod 204, 206. The use of an eccentric cam surface to control the operation of the cutting blade 201, together with the shape of the cutting throat defined by mandrel 189 insures a smooth, even and sharp cut through the absorbent pad web 15, to define individual pads 22.

Details of the pad conveyor section 28 of Figure 1A are shown in Figures 10 and 11. The pad conveyor includes a pair of rotating shafts 281, 282 which extend laterally across the direction of manufacture, and support plural vinyl conveyor straps 283 along the manufacturing direction. As is depicted in Figure 7, the conveyor webs 283 are extended into close proximity to the throat of the pad cutter 20.

A pair of chain drives 284 are supported parallel to and underneath the webbing 283, each chain drive 284 spaced between two of the webbing strips. The chain drives are supported by shaft 285 and an eccentric drive 287 (Figure 11). Plural straightening dogs 288 are dimensioned vertically to extend upwardly from the respective chain drives 284 and above the level of the webbing strips 283. As is depicted in Figure 10, the eccentric drive 287 imparts a "slow-fast-slow" pace to the movement of the straightening dogs, in order that the dogs may first extend upwardly between adjacent strips 283, then be speeded up to engage the backside of a corresponding pad 22 to straighten the position of any misaligned pad across the lateral direction, and then slow down and back away from the back edge of the corresponding pad, before rotating underneath the chain drive as shown in Figure 11. Of course, the pad 22 is then discharged from the pad conveyor 28 on the right hand side of Figure 10, and progresses to the combining section 32 (Figure 1A).

As shown in Figure 13, a web of the elastic sheet 40 (i.e., urethane foam or polyethylene) is passed into a knife roller 381 having knife blades 382, at which point the web of elastic sheet is cut into individual sheet segments 40. the knife roller 381 cuts the elastic sheet lengths 40, and vacuum chucks 383 on anvil 384 carries the elastic sheet lengths 40 to a transfer roller 385.

Noting both Figures 12 and 13, at an appropriate transfer point, the individual elastic sheets 40 are received along the surface of the transfer roller 385 which also includes vacuum chicks 386. The vacuum chucks 386 hold the individual elastic lengths 40 across the transfer surface of the transfer roller 385, until such time as the individual sheets 40 are fed into an entry point 387 of stretching rollers 388, 389. As is particularly shown in Figure 12, the crosswise dimension of the transfer surface of the transfer roller 385 is substantially less than the lengthwise dimension of each elastic sheet 40, and is approximately equal to or less than the inner spacing between the stretching rollers 388, 389 at the entry point 387.

The stretching rollers 388, 389 are mounted on respective hubs 390, 391, each of which may be individually adjusted at an outwardly skewed angle from the entry point 387 to an exit point 392. Each rotating stretching roller 388, 389 also includes a corresponding peripheral groove 393, 394 across which passes a corresponding smooth surface gripping belt 395, 396, each of which is dimensioned to fit within the corresponding peripheral groove 393, 394. As each gripping belt 395, 396 approaches the periphery of the corresponding roller 388, 389 at the entry point 387, an adjacent one of the elastic sheets 40 are picked up at the transfer point by the gripping belts and pinched into the corresponding groove 393, 394. As the skewed rollers 388, 389 are rotated, the gripping belts hold the sheets in place, and the skewed angle effectuates the desired degree of stretching. Thereafter, as is shown in Figure 13, the individual sheets 40 are discharges at area 392 onto the web of the outer non-woven layer 34.

The edge folding section 52 includes a pressure roller 522, under which the combined web assembly including the outer non-woven layer 34, liner (not depicted in Figure 14), absorbent pad 22 and inner non-woven layer 50 are all passed. As is illustrated in Figure 14, the pressure roller 522 includes inwardly tapered, low friction surfaces which are dimensioned to engage only the extended edge portions 33, 35 of the outer non-woven layer 34. Because the tension of the web is relieved by the taper 526 on the in caps 524, the edge portions 33, 35 are lifted upwardly. Thereafter, the web is passed across edge folders 528, each of which includes a tapered fold surface 530 for receiving the elevated edge portions 33, 35. A glue joint 532 is then directed across the outer edge of the inner non-woven layer 50, for receiving the folded edges 33, 35 of the outer non-woven layer 34.

As discussed above, the seam weld section 60 (Figure 1B) utilizes an ultrasonic welder including weld head 602 having a face 604 with protruding weld pins 606. In accordance with the present invention, the pins 606 are sharpened to a sufficient degree to insure penetration through the entire web assembly, including the outer non-woven layer 34, elastic sheet 40, liner 48 and inner non-woven layer 50. Further in accordance with the present invention, the ultrasonic welder is operated in conjunction with a rotating anvil 607 having an anvil face 608 which is heated by an internal heating coil 609 to a temperature which is slightly below the melting temperature of the polyvinyl liner 48, and of course below the ignition temperature of the inner and outer non-woven layers 34, 50. It has been found that this particular arrangement shown in Figure 16 provides desirable seam welds in a facile manner.

A second form of the seam welding section is depicted in Figures 17A-C. In this arrangement, the seam welding section includes opposing pairs of rotating drive wheels 704, 706 and 716, 718. Each pair of drive wheels is pivotally connected respectively at 710, 712 and 722, 724 to a corresponding drive rod 708, 720. (As shown in Figure 17B, each of the drive wheels is in turn rotated by a corresponding shaft and gear arrangement, including corresponding gears 795, 707 and 717, 719, with each the drive gear being driven by a respective pinion 703, 715).

A conventional ultrasonic horn 714 is mounted on the forward extremity of the drive rod 708, and a conventional ultrasonic anvil 726 is mounted at the extremity of the second drive rod 720. In accordance with the present invention, the anvil is coupled to a back plate 730 via an air bladder 728 which is capable of being alternately inflated and deflated from an air supply 729. This construction permits the anvil 726 to be inflated outwardly toward the horn 714, and also imparts a significant degree of "give" to the anvil 726 via the air bladder 728.

In operation, rotation of each pair of drive wheels 704, 706 and 716, 718 results in the reciprocal movement of the corresponding drive rod 708, 720 toward the machine web, represented by arrow 702 in Figures 17A and 17C. It will of course be appreciated by those skilled in the ultrasonic welding art that without the presence of the compressible bladder 728, there is only one tangential point where the ultrasonic horn 714 and the anvil 726 may come in contact with each other, in order to avoid any damage to either part. However, with the provision of the inflatable bladdder 728, the anvil 726 is imparted with a significant degree of "give" or compressibility, thereby permitting the horn 714 and the anvil 726 to be in contact with each other for a much greater period of time during movement of the web (represented by arrow 702) along the direction of travel. This results in a much better forming of weld seams like seam 62 and 64 in Figure 2B. This greater dimension of contact is represented by dimension D in Figure 17C.

In conclusion, there has been disclosed above a method and apparatus for manufacturing disposable garments according to the invention and representing a preferred embodiment. The disposable garments 70 and 90 of Figures 3-6, in particular provide a cuff which extends inwardly from the plane of the leg hole, to provide a liquid barrier at least in the region of the lower portion of the leg opening. This is achieved utilizing an elastic sheet which may be stretched to a sufficient degree to insure achieving the desired rolled cuff feature, and in a manner which lends itself to high speed, low cost manufacturing techniques. It will be apparent, however, that various modification and changes may be made in the above described method and apparatus, and in the manufactured product without departing from the invention herein described and hereinafter claimed.

## Claims

1. A disposable garment of the kind comprising front and back panels and an integral crotch portion of water-impervious sheet material together defining a waist opening and a pair of elasticated leg openings (56) for resilient engagement around the leg of the wearer, characterised in that the garment comprises at least one sheet (40) of elasticated material secured to the inside surface of the garment in surrounding relationship to at least a portion of the periphery of each of the leg openings (56), the said elasticated sheet or sheets (40) being secured to the inside of the garment in a stretched condition such that upon contraction the periphery of each leg opening is drawn into a curved elasticated cuff (80,82).

2. A disposable garment according to claim 1 characterised in that the elasticated sheet or sheets (40) extends fully to the peripheral edge of the leg openings (56).

3. A disposable garment according to claim 1 or 2 characterised in that the said elasticated sheet(s) (40) extend a distance of at least 1.27 cms (0.5 in) inwardly from each leg opening.

4. A disposable garment according to any one of claims 1 to 3, wherein the waist opening (72) is also elasticated (46).

5. A disposable garment according to any one of claims 1 to 4, wherein the elasticated sheet(s) (40) extend wholly around the periphery of each leg opening (56).

6. A disposable garment according to any one of claims 1 to 4, wherein the elasticated sheet(s) (40) extend around the lower curved portion only of each leg opening (56).

7. A disposable garment according to any one of the preceding claims, wherein the direction of stretch applied to the elasticated sheet(s) (40) prior to the attachment thereof inside the garment is parallel to the leg opening about the lower curved portion of each leg opening (56) and in a direction towards the waist opening about the upper portion of the leg opening.

8. A disposable garment according to any one of the preceding claims, further comprising a pair of tearable side seams (62,64) extending from the waist opening (72) to each leg opening (56).

9. A disposable garment according to any of the preceding claims, further comprising an absorbent pad (22) secured inside the garment in the crotch area between the leg openings (56).

10. A disposable garment according to claim 9, further comprising a thin, liquid permeable inner liner (50) secured inside the garment and overlying the pad (22) and the elasticated sheet(s) (40) about each leg opening (56).

11. A disposable garment according to any one of the preceding claims, further comprising a second elasticated sheet (93) or elasticated strips (92) secured inside the crotch area of the garment in a prestretched condition and transverse to the leg holes (56) and effective to form a gathering of the material forming the garment in the crotch area between the leg holes (56).

12. A disposable garment according to claim 11, wherein said transverse elasticated strips (92) also extend around the periphery of each leg hole (56).

13. A disposable garment according to any one of claims 1-12 wherein the elasticated sheet(s) (40) is or are secured inside the garment whilst under an applied stretch of about 55.86 g per cm (5 oz. per lined inch) for each 2.54 cms (1 in.) of cross-width.

14. A method for the manufacture of disposable garments of the kind comprising a sheet (34) of flexible material impervious to liquids folded to form front and back panels defining a waist opening (72), a pair of leg openings (56) and a crotch area between said leg openings, each leg opening having a lower curved portion adjacent the crotch area and an upper curved portion extending towards the waist opening, characterised by the steps of stretching a sheet of sheets (40) of elasticated material, affixing the or each sheet of elasticated material (40) whilst in the stretched condition to the sheet (34) of water impermeable material in the regions thereof of which define said front and back panels, said sheet or sheets (40) extending around at least the lower curved portion of each leg opening (56), and allowing the or each sheet of elasticated material to relax thereby to draw at least the lower portion of the periphery of the corresponding leg opening into a rolled cuff inwardly from the plane of that opening.

15. A method according to claim 14, characterised in that the or each stretched elasticated sheet (40) is affixed to said front and back panels so that an edge of each sheet is aligned exactly with the periphery of the corresponding leg opening.

16. A method according to claim 15, characterised by the steps of affixing said sheet(s) (40) of elasticated material to the front and back panels regions of said water-impermeable sheet material (34) prior to the formation of the leg holes (56) therein, and then simultaneously cutting the leg openings (56) in the front and back panels and through the preaffixed sheet(s) (40) of elasticated material.

17. A method according to claim 14, 15 or 16, characterised in that the or each elasticated sheet (40) is affixed to said front and back panels in a position such that each sheet extends inwardly from the edge of the corresponding leg opening a distance of at least 1.72 cms (0.5 in.).

18. A method according to any one of claims 14-17, characterised in that prior to affixing the elasticated sheet(s) (40) to the front and back panels, the or each sheet is stretched to about 55.86 g per cm (5 oz. per lined inch) for each 2.54 cms (1 in.) of cross-width.

19. A method according to any one of claims 14-18, further comprising the step of forming tearable side seams (62,64) between the front and back panels and extending from the waist opening (72) to the upper portion of each leg opening (56).

20. A method according to any one of claims 14-19, further comprising the step or steps of affixing an absorbent pad (22) to said front and back panels and between said leg openings (56).

21. A method according to any one of claims 14-20 further comprising the steps of affixing a second elasticated sheet (93) or elasticated strips (92) in a prestretched condition in the crotch area of the garment, the direction of stretch being transverse to the leg openings (56) and allowing that second elasticated sheet (93) to relax thereby to form a gathering of said sheet material (34) in said crotch area between the two leg openings (56).

22. A method according to claim 14 characterised by the steps of
i) advancing a continuous web of water impermeable sheet material (34) in an open unfolded configuration along a manufacturing path and in the longitudinal direction of the web;
ii) forming a series of individual absorbent pads (22);
iii) affixing those pads to the advancing web (34) at regularly spaced intervals and in a direction transverse to the length of the web;
iv) forming and prestretching a series of individual sheets (40) of elasticated sheet material;
v) affixing those prestretched sheets (40) of elasticated sheet material in the stretched condition to the advancing web of sheet material (34) in the spaces between said absorbent pads and with the direction of stretch oriented transverse to the web;
vi) laying and securing continuous strips (46) of elasticated strip material along the opposite longitudinal edges of the advancing web;
vii) cutting through holes (56) through said prestretched elasticated sheets (40) when affixed to the advancing web of sheet material (34) and through the underlying web to form said leg holes;
viii) folding the advancing web (34) longitudinally about its centre line, following the attachment of said elasticated strips (46), said pads (22), said elasticated sheets (40) and the cutting of the said assembly to form said leg holes, thereby to align said elasticated strips (46) one with the other;
ix) forming a pair of parallel seams (62,64) at intervals along the length of the folded web and extending perpendicularly from the superimposed longitudinal edges of the folded web to each of the adjacent leg holes (56), and joining the folded plies of said water-impermeable sheet material (34) one to the other; and
x) transversely chopping the folded web between each pair of parallel seams (62,64) into a series of discrete units, each comprising a section of said water impermeable sheet material folded upon itself to form said front panels, an elasticated waist opening, a pair of leg openings, an absorbent pad secured to said front and back panels between the leg openings and a pair of side seams extending from each leg opening to the waist opening.

23. A method according to claim 22, wherein the web of water-impermeable sheet material is a composite structure comprising an underlying layer of non-woven fabric and a water-impermeable liner bonded to the surface of the non-woven fabric, and where the method further comprises the initial step of bonding a continuous web of water-impermeable lining material to an advancing continuous web of non-woven fabric prior to the positioning and attachment of the absorbent pads to the composite structure.

24. A method according to claim 22 or 23, comprising the further step of laying a further continuous web (50) of non-woven lining material on top of the advancing web (34) after the positioning thereon of said absorbent pads (22) and said elasticated sheets (40) but prior to the formation of said leg holes (56).

25. A method according to any one of claims 22-24, comprising the further steps of forming and prestretching a second series of individual elasticated sheets (93), or elasticated strips (92) attaching those elasticated sheets (93) or strips (92) in the stretched condition centrally to the advancing web, prior to said folding step, and under or over said pads (22) and under or ever said elasticated sheets (40), the direction of stretch in that case being parallel to the length of the web.

26. A method according to claim 25, wherein said further step or steps comprise longitudinally prestretching individual elasticated strips (92) and affixing those elasticated strips (92) in the stretched condition centrally of the advancing web and parallel to the length thereof at least in the uninterrupted portion of the advancing web between the leg holes, those strips (92) furthermore having a length such that the opposite ends thereof extend at least partially around the periphery of the holes which are or are to be cut in the advancing web to form the said leg holes.

27. A method according to any one of claims 22-26 characterised in that the said elasticated sheets (40) and in the case of claim 26, the elasticated sheets (93) or said strips (92) are applied to the advancing web (34) under an applied stretch of the order of about 55.86 g per cm (5 oz. per lined inch) for each 2.54 cms (1 in.) of cross-width.

28. A method for making disposable garments, characterised by the steps of:
conveying a carrier layer having opposing edges along a direction of manufacture;
disposing plural elongated absorbent pads in spaced relation on the carrier layer between the opposing edges within the direction of elongation extending generally lateral to the direction of manufacture;
folding the carrier layer and the pads generally into two halves along a fold line intermediate between the opposing edges and generally parallel with the direction of manufacture;
joining the two halves together along plural seam lines, each seam line extending generally lateral to the direction of manufacture between adjacent pads, and intersecting a leg hole and the opposing sides; and
severing the carrier layer into individual garment units between adjacent seam lines.

29. A method according to claim 28, characterised by one or more of the following features:
i) that the hole forming step comprises cutting the carrier layer centrally between the opposing edges;
ii) that the joining step comprises the steps of: contacting the carrier layer with a heated anvil to elevate the temperature of the carrier layer in the area where the seams are to be formed; and
engaging the carrier layer with an ultrasonic welder in the area where the seam lines are to be formed while the temperature of the carrier layer is elevated;
iii) prior to the absorbent pad disposing step, the step of forming the absorbent pads from an absorbent core material and a liquid permeable tissue, said absorbent pad forming step preferably comprising the steps of:
conveying a continuous layer of the absorbent core material along a conveying direction;
folding the liquid permeable tissue around the absorbent core layer around the absorbent core layer during movement along the conveying direction; and
cutting the permeable tissue-absorbent core combination at a cutter into individual pads having the direction of elongation generally lateral to the conveying direction, the individual pad cutting step preferably comprising the steps of:
intermittently feeding the permeable tissue-absorbent core combination into the cutter; and
reciprocating a cutting blade through the permeable tissue-absorbent core combination along a cutting plane at the cutter, which cutting plane is generally lateral to the conveying direction, the intermittent feeding step preferably comprising the steps of:
engaging the permeable tissue-absorbent core combination with an indexing conveyor preferably by means of a vacuum; and
intermittently stepping the indexing conveyor toward the cutter;
the pad forming step preferably further comprising:
the step of continuously feeding the permeable tissue-absorbent core combination across the indexing conveyor, preferably whilst elevating the permeable tissue-absorbent core combination to intermittently buckle between the accumulator and the indexing conveyor, the degree of buckle between the accumulator and the indexing conveyor preferably being controlled;
the pad forming step preferably further comprising:
receiving the individual pads as an input to a pad conveyor immediately downstream from the cutter and moving each pad across the pad conveyor to an output thereof;
engaging each pad during movement along the pad conveyor for correcting any lateral misalignment of the direction of elongation of each pad relative to the conveying direction; and thereafter disposing each aligned pad onto the carrier layer downstream from the output of the pad conveyor, the said engagement step preferably comprising extending a pair of spaced straightening dogs through the conveying station and increasing the speed of the dogs relative to movement of each pad to engage downstream elongated side of each pad to thereby properly align any misaligned pad, the speed of the dogs relative to the movement of the pads preferably being reduced upstream from the output of the pad conveyor;
iv) the step of forming the carrier layer prior to the step of disposing the elongated absorbent pads onto the carrier layer, the carrier layer forming step preferably comprising conveying a liquid impervious liner along the director of manufacture, adhering each pad to the liner; conveying non-woven inner and outer liquid permeable layers along the direction of manufacture with the liner and each pad between the inner and outer layers; and adhering the inner and outer layers to opposing sides of the barrier layer, to form the carrier layers, preferably folded inwardly and adhered to the edge portion of the outer non-woven layer across the edge of the inner non-woven layer;
v) the folding step comprises the steps of:
providing a fold roller having outwardly tapered ends, the lateral dimension of each tapered end corresponding to the dimension of the edge portions;
passing the carrier under the fold roller under sufficient tension to cause each edge portion of the outer non-woven layer to conform to the shape of the adjacent end taper and thereby exit the roller with the edge portions of the outer non-woven layer slightly elevated relative to the plane of the carrier layer; and
engaging the raised edge portions immediately downstream from the fold roller and folding and adhering the raised edge portions to the edge of the inner non-woven layer;
vi) elasticizing the waistband; and
vii) elasticizing the edges of the leg holes.
